**Europäisches Patentamt**

**European Patent Office**

**Office européen des brevets**

(19)

(11) **EP 0 922 739 B1**

(12) **EUROPÄISCHE PATENTSCHRIFT**

(45) Veröffentlichungstag und Bekanntmachung des Hinweises auf die Patenterteilung:
**04.08.2004 Patentblatt 2004/32**

(51) Int Cl.⁷: **C09J 7/04**, C09J 7/02, A61L 15/58

(21) Anmeldenummer: **98122139.3**

(22) Anmeldetag: **25.11.1998**

(54) **Selbstklebend ausgerüstetes Trägermaterial**

Backing material equipped with self-adhesive

Support équipé avec matériau auto-adhésif

(84) Benannte Vertragsstaaten:
**AT DE ES FR GB IT NL SE**

(30) Priorität: **12.12.1997 DE 19755222**

(43) Veröffentlichungstag der Anmeldung:
**16.06.1999 Patentblatt 1999/24**

(73) Patentinhaber: **Beiersdorf Aktiengesellschaft 20245 Hamburg (DE)**

(72) Erfinder:
• **Himmelsbach, Peter 21614 Buxtehude (DE)**
• **Jauchen, Peter 22455 Hamburg (DE)**

(56) Entgegenhaltungen:
**EP-A- 0 826 380**      **EP-A- 0 918 080**
**DE-A- 19 620 107**     **US-A- 4 024 312**

**Beschreibung**

Die Erfindung betrifft ein zumindest einseitig selbstklebend ausgerüstetes Trägermaterial, das partiell beschichtet.

Klebend beschichtete Trägersysteme, die auf Zug am Träger entkleben, sind bekannt. Sie basieren in der Regel auf elastischen Systemen, welche häufig Laminate darstellen.

DE-OS 27 28 346 beschreibt ein solches Laminat, welches aus einer dehnfähigen Folie und einer Klebemasse auf Basis von A-B-A-Blockmischpolymerisaten besteht. Das gesamthaftende Laminat ist durch Strecken leicht von dem Untergrund zu lösen.

Die Klebmasse ist stets vollflächig aufgetragen. Ein solche vollflächige Auftragsweise ist aber mit einer Vielzahl von Nachteilen verbunden, die insbesondere den Einsatz im medizinischen Bereichen stark einschränken.

Eine vollflächige Auftragsweise führt dazu, daß hohe Mengen an Klebmasse benötigt werden, was den Herstellungsprozeß und damit auch das Endprodukt verteuert. Des weiteren ist es wichtig, daß das mit der Klebmasse beschichtete Trägermaterial nach der Applizierung auf der Haut weiterhin luft- und wasserdampfdurchlässig ist, damit beispielsweise Schweiß von der Haut abtransportiert werden kann. Diese Eigenschaft weist ein vollflächig beschichteter Träger nicht oder nur sehr eingeschränkt auf.

DE-OS 195 31 696 beschreibt ein Klebefolienlaminat, welches aus einem dehnfähigen Träger und einer Acrylatklebemasse hergestellt wird.

WO 95/06691 offenbart ein wiederablösbares Klebeband, das einen Schaum als Trägermaterial aufweist.

WO 92/11333 beschreibt ein zerstörungsfrei entfernbares System, welches eine hohe Reißfestigkeit besitzt. Eine partielle Beschichtung ist aber ebenfalls nicht beschrieben

Es ist ferner bekannt, Selbstklebemassen auf Trägermaterialien für rückstandsfrei wiederablösbare Flächengebilde und/oder medizinische Anwendungen nicht nur vollflächig, sondern auch rasterpunktförmig, beispielsweise durch Siebdruck (DE-PS 42 37 252), wobei die Klebstoffpünktchen auch unterschiedlich groß und/oder unterschiedlich verteilt sein können (EP-PS 353 972), oder durch Tiefdruck von in Längs- und Querrichtung zusammenhängenden Stegen aufzubringen (DE-PS 43 08 649). Ein durch Dehnung des jeweiligen Trägers in Orientierung des Trägers hervorgerufenen Ablösungseffekt (Strippeffekt) ist aber in den aufgeführten Dokumenten nicht beschrieben.

Aus dem Stand der Technik sind einseitig selbstklebende Trägermaterialien (EP-A-0826380) und partiell beschichtete Trägermaterialien (EP-A-0918080 und DE-A-19620107) sowie deren Verwendung für medizinische Produkte bekannt. In allen drei Druckschriften wird keinerlei Aussage über die Ablösbarkeit und das Entklebungsverfahren der Träger von der Haut offenbart. Obwohl einerseits vollflächig selbstklebend beschichtete dehnbare Trägermaterialien und andererseits partiell selbstklebende beschichtete Trägermaterialien bekannt sind, die sich vom Untergrund ablösen lassen, findet sich jedoch kein Hinweis im Stand der Technik, dass die Ablösbarkeit bis hin zur vollständigen Entklebung durch Ziehen dieser besonderen Trägermaterialien in Richtung der Verklebungsebene, insbesondere schmerzfrei, möglich ist.

Einen gravierenden Nachteil weisen alle zuvor beschriebenen Systeme auf, da sie vollflächig beschichtet worden sind. Hierdurch wird zum Wiederablösen eine vergleichsweise hohe Kraft benötigt, wodurch die Grenze der Festigkeit der Trägermaterialien erreicht wird.

Weiter nachteilig sind für diese Trägermaterialien, daß bei einer vollflächigen Beschichtung die verwendeten Systeme ihre Elastizität verlieren können, wenn die verwendeten Klebesysteme eine geringere Elastizität aufweisen.

Die zuvor genannten Klebemassen können für die Verarbeitung in einer Trägermatrix vorliegen. Als Trägermatrix werden gängige organische oder anorganische Lösemittel oder Dispergiermittel verstanden.

Systeme ohne Trägermatrix werden als 100 %-Systeme bezeichnet und sind ebenfalls nicht unbekannt. Sie werden im elastischen oder thermoplastischen Zustand verarbeitet. Eine gängige Verarbeitungsweise ist die Schmelze. Auch solche Heißschmelzklebemassen sind im Stande der Technik bereits vorbeschrieben. Sie basieren auf natürlichen oder synthetischen Kautschuken und/oder anderen synthetischen Polymeren.

Aufgrund ihrer hohen Härte ist für solche 100 %-Systeme die Hauthaftung problematisch.

Vorteilhaft an den 100 %-Systemen ist, daß bei ihnen verfahrenstechnisch ein Entfernen der Trägermatrix, d.h. der Hilfsmittel, vermieden wird, wodurch sich die Verarbeitungsproduktivität steigert und sich gleichzeitig der Maschinen- und Energieaufwand reduziert. Weiter wird so ein Verbleiben von Reststoffen der Trägermatrix reduziert. Dieses begünstigt wiederum die Senkung des allergenen Potentials, bei einer Verwendung speziell für die Hautverklebung.

Aufgabe der Erfindung war es, die aus dem Stand der Technik bekannten Nachteile zu vermeiden und ein zumindest einseitig selbstklebendes Trägermaterial zur Verfügung zu stellen, das sich durch Dehnung einfach und insbesondere bei seiner Verwendung als medizinisches Produkt schmerzfrei nach der Applizierung auf der Haut wieder ablösen läßt.

Gelöst wird diese Aufgabe durch ein Verfahren zur Verwendung eines zumindest einseitig selbstklebend ausgerüsteten Trägermaterials gekennzeichnet durch die Schritte

- Herstellung medizinischer Produkte,
- Applikation dieser auf der menschlichen Haut und
- Ablösung dieser durch Ziehen in Richtung der Verklebungsebene vom Untergrund,

wobei das Trägermaterial partiell mit einer Selbstklebemasse beschichtet ist, eine Klebkraft auf der Trägerrückseite von mindestens 0,5 N/cm und bei einer Zugbelastung von 10 N/cm eine Dehnung von 15% bis 3000% aufweist.

Vorzugsweise weist das Trägermaterial bei einer Zugbelastung von 10 N/cm eine Dehnung von 20% bis 1000% auf.

Vorteilhaft zeigte sich, daß bei der Verwendung von wasserdampf- oder luftdurchlässigen Trägermaterialien, die klebend ausgerüsteten Produkte auch bei hohen Masseaufträgen von größer 15 g/m$^2$ die Durchlässigkeit enorm hoch war.

Als Trägermaterialien eignen sich dehnbaren Flächengebilde aus synthetischen und natürlichen Rohstoffen. Bevorzugt sind Trägermaterialien die nach Applikation der Selbstklebemasse so eingesetzt werden können, daß sie Eigenschaften eines funktionsgerechten Verbandes erfüllen. Beispielhaft sind Textilien wie Gewebe, Gewirke, Gelege, Vliese, Laminate, Netze, Folien, Schäume und Papiere aufgeführt, welche eine Dehnbarkeit von mindestens 10 % bei einer Belastung von 10 N/cm aufweisen.

Geeignet sind darüber hinaus auch die Kombinationen der genannten Materialien.

Weiter können diese Materialien vor- bzw. nachbehandelt werden. Gängige Vorbehandlungen sind Corona und Hydrophobieren; geläufige Nachbehandlungen sind Kalandem, Tempern, Kaschieren, Stanzen und Eindecken, UV-/IR Bestrahlung oder Elektronenbestrahlung.

Als Selbstklebemassen für die Beschichtung lassen sich vorteilhafterweise thermoplastische Heißschmelzklebemassen einsetzen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich.

Ihr Erweichungspunkt sollte höher als 50 °C liegen, da die Applikationstemperatur in der Regel mindestens 90 °C beträgt, bevorzugt zwischen 120 °C und 150 °C beziehungsweise 180 °C und 220 °C bei Silikonen. Gegebenenfalls kann eine Nachvernetzung durch UV- oder Elektronenstrahlen-Bestrahlung angebracht sein.

Insbesondere Heißschmelzklebemassen auf Basis von Blockcopolymeren zeichnen sich durch ihre vielfältige Variationsmöglichkeiten aus, denn durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung gewährleistet, insbesondere mit der Haut auch an kritischen Stellen des menschlichen Bewegungsapparates.

Die hohe Scherfestigkeit der Heißschmelzklebemasse wird durch die hohe Kohäsivität des Polymeren erreicht. Die gute Anfaßklebrigkeit ergibt sich durch die eingesetzte Palette an Klebrigmachern und Weichmachern.

Bevorzugt basiert die Heißschmelzklebemasse auf Blockcopolymeren, insbesondere A-B-, A-B-A-Blockcopolymere oder deren Mischungen. Die harte Phase A ist vornehmlich Polystyrol oder dessen Derivate, und die weiche Phase B enthält Ethylen, Propylen, Butylen, Butadien, Isopren oder deren Mischungen, hierbei besonders bevorzugt Ethylen und Butylen oder deren Mischungen.

Polystyrolblöcke können aber auch in der weichen Phase B enthalten sein, und zwar bis zu 20 Gew.-%. Der gesamte Styrolanteil sollte aber stets niedriger als 35 Gew.-% liegen. Bevorzugt werden Styrolanteile zwischen 5% und 30%, da ein niedrigerer Styrolanteil die Klebemasse anschmiegsamer macht.

Insbesondere die gezielte Abmischung von Di-Block- und Tri-Blockcopolymeren ist vorteilhaft, wobei ein Anteil an Di-Blockcopolymeren von kleiner 80 Gew.-% bevorzugt wird.

In einer vorteilhaften Ausführung weist die Heißschmelzklebemasse die nachfolgend angegebene Zusammensetzung auf:

| | |
|---|---|
| 10 Gew.-% bis 90 Gew.-% | Blockcopolymere, |
| 5 Gew.-% bis 80 Gew.-% | Klebrigmacher wie Öle, Wachse, Harze und/oder deren Mischungen, bevorzugt Mischungen aus Harzen und Ölen, |
| gegebenenfalls weniger als 60 Gew.-% | Weichmacher, |
| weniger als 15 Gew.-% | Additive, |
| weniger als 5 Gew.-% | Stabilisatoren. |

Die als Klebrigmacher dienenden aliphatischen oder aromatischen Öle, Wachse und Harze sind bevorzugt Kohlenwasserstofföle, -wachse und -harze, wobei sich die Öle, wie Paraffinkohlenwasserstofföle, oder die Wachse, wie Paraffinkohlenwasserstoffwachse, durch ihre Konsistenz günstig auf die Hautverklebung auswirken. Als Weichmacher finden mittel- oder langkettige Fettsäuren und/oder deren Ester Verwendung. Diese Zusätze dienen dabei der Einstellung der Klebeeigenschaften und der Stabilität. Gegebenenfalls kommen weitere Stabilisatoren und andere Hilfsstoffe zum Einsatz.

Ein Füllen der Klebemasse mit mineralischen Füllstoffen, Fasern, Mikrohohl- oder -vollkugeln ist möglich.

Die Heißschmelzselbstklebemasse weist einen Erweichungspunkt auf oberhalb von 70 °C, bevorzugt 95 °C bis 120 °C.

Die Heißschmelzselbstklebemassen sind vorzugsweise so eingestellt, daß sie bei einer Frequenz von 0,1 rad/s eine dynamisch-komplexe Glasübergangstemperatur von weniger als 5 °C, bevorzugt von -3 °C bis -30 °C, ganz besonders bevorzugt von -9 °C bis -25 °C, aufweisen.

Insbesondere an medizinische Produkte werden hohe Anforderungen bezüglich der Klebeeigenschaften gestellt. Für eine ideale Anwendung sollte die Heißschmelzklebemasse eine hohe Anfaßklebrigkeit besitzen. Die funktionsangepaßte Klebkraft auf der Haut und auf der Trägerrückseite sollte vorhanden sein. Weiterhin ist, damit es zu keinem Verrutschen kommt, eine hohe Scherfestigkeit der Heißschmelzklebemasse notwendig.

Durch die erfindungswesentliche gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut und der Trägerrückseite erreicht. Die hohe Scherfestigkeit der hier eingesetzten Selbstklebemasse wird durch die hohe Kohäsivität des Blockcopolymeren erreicht. Die gute Anfaßklebrigkeit ergibt sich durch die eingesetzte Palette an Klebrigmachern und Weichmachern.

Die Produkteigenschaften wie Anfaßklebrigkeit, Glasübergangstemperatur und Scherstabilität lassen sich mit Hilfe einer dynamisch-mechanischen Frequenzmessung gut quantifizieren. Hierbei wird ein schubspannungsgesteuertes Rheometer verwendet.

Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur die Heißschmelzselbstklebemasse zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient ($Q = \tan \delta$) zwischen dem Verlustmodul (G" viskoser Anteil) und dem Speichermodul (G' elastischer Anteil) ermittelt.

$$Q = \tan \delta = G''/G'$$

Für das subjektive Empfinden der Anfaßklebrigkeit (Tack) wird eine hohe Frequenz gewählt sowie für die Scherfestigkeit eine niedrige Frequenz.

Eine hoher Zahlenwert bedeutet eine bessere Anfaßklebrigkeit und eine schlechtere Scherstabilität.

Die Glasübergangstemperatur ist die Temperatur, bei der amorphe oder teilkristalline Polymere vom flüssigen oder gummielastischen Zustand in den hartelastischen oder glasigen Zustand übergehen oder umgekehrt (Römpp Chemie-Lexikon, 9. Aufl., Band 2, Seite 1587, Georg Thieme Verlag Stuttgart - New York, 1990). Er entspricht dem Maximum der Temperaturfunktion bei vorgegebener Frequenz.

Besonders für medizinische Anwendungen ist ein relativ niedriger Glasübergangspunkt notwendig.

| Bezeichnung | $T_G$ niedrige Frequenz | Anschmiegsamkeit niedrige Frequenz/RT | Anfaßklebrigkeit hohe Frequenz/RT |
|---|---|---|---|
| Heißschmelzklebemasse A | -12 ± 2 °C | $\tan \delta = 0,08 \pm 0,03$ | $\tan \delta = 0,84 \pm 0,03$ |
| Heißschmelzklebemasse B | -9 ± 2 °C | $\tan \delta = 0,32 \pm 0,03$ | $\tan \delta = 1,70 \pm 0,03$ |

Bevorzugt werden erfindungsgemäß Heißschmelzklebemassen, bei denen das Verhältnis des viskosen Anteils zum elastischen Anteil bei einer Frequenz von 100 rad/s bei 25 °C größer 0,7 ist, bevorzugt 1,0 bis 5,0, oder Heißschmelzselbstklebemassen, bei denen das Verhältnis des viskosen Anteils zum elastischen Anteil bei einer Frequenz von 0,1 rad/s bei 25 °C kleiner 0,4 ist, bevorzugt zwischen 0,35 und 0,02, ganz besonders bevorzugt zwischen 0,3 und 0,1.

Vorteilhaft insbesondere für die Verwendung bei medizinischen Produkten ist weiterhin, wenn die Heißschmelzklebemasse partiell auf dem Trägermaterial aufgetragen ist, beispielsweise durch Rasterdruck, Thermosiebdruck, Thermoflexodruck oder Tiefdruck, denn im Vollstrich klebend ausgerüstete Trägermaterialien können leicht bei der Applikation mechanische Hautirritationen hervorrufen.

Der partielle Auftrag ermöglicht durch geregelte Kanäle die Abführung des transepidermalen Wasserverlustes und verbessert das Ausdampfen der Haut beim Schwitzen insbesondere bei der Verwendung von luft- und wasserdampfdurchlässigen Trägermaterialien. Hierdurch werden Hautirritationen, die durch Stauungen der Körperflüssigkeiten hervorgerufen werden, vermieden. Die angelegten Abführungskanäle ermöglichen ein Ableiten auch unter Verwendung eines mehrlagigen Verbandes.

Zudem ermöglicht bei Trägermaterialien mit hohem Flächengewicht und geringer Dehnung, wie sie beispielsweise für Kurzzugbinden üblich sind, die partielle Beschichtung erst das Wiederablösen durch Dehnung, welches trotz der hohen Klebekraft dieser Klebebindensysteme recht schmerzfrei ist.

Bevorzugt wird der Auftrag in Form von polygeometrischen Kalotten und ganz besonders von solchen Kalotten, bei denen das Verhältnis Durchmesser zu Höhe kleiner 5:1 ist. Weiterhin ist auch der Aufdruck anderer Formen und Muster auf dem Trägermaterial möglich, so beispielsweise ein Druckbild in Form alphanumerischer Zeichenkombinationen oder Muster wie Gitter, Streifen, des weiteren Kumulate der Kalotten und Zickzacklinien.

Ferner kann sie beispielsweise auch aufgesprüht sein, was ein mehr oder weniger unregelmäßiges Auftragsbild ergibt.

Die Selbstklebemasse kann gleichmäßig auf dem Trägermaterial verteilt sein, sie kann aber auch funktionsgerecht für das Produkt über die Fläche unterschiedlich stark oder dicht aufgetragen sein.

Das Prinzip des Thermosiebdrucks besteht in der Verwendung einer rotierenden beheizten, nahtlosen, trommelförmigen perforierten Rundschablone, die über eine Düse mit der bevorzugten Heißschmelzselbstklebemasse beschickt wird. Eine speziell geformte Düsenlippe (Rund- oder Vierkantrakel) preßt die über einen Kanal eingespeiste Heißschmelzklebemasse durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Ausbildung der kleinen Klebstoffkalotten geschieht dabei nach folgendem Mechanismus:

Der Düsenrakeldruck fördert die Heißschmelzklebemasse durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Selbstklebemasse und die innere Kohäsion des Hotmelts wird von der auf dem Träger bereits haftenden Basis der Kalotten der in den Löchern begrenzte Vorrat an Heißschmelzklebemasse konturenscharf abgezogen beziehungsweise durch den Rakeldruck auf den Träger gefördert.

Nach Beendigung dieses Transportes formt sich, abhängig von der Rheologie der Heißschmelzklebemasse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Selbstklebemasse ab.

Bei der Siebschablone im Thermosiebdruck kann das Steg/Loch-Verhältnis kleiner 3:1 sein, bevorzugt kleiner oder gleich 1:1, insbesondere gleich 1:3.

Der beschriebene Bildungsmechanismus der Kalotten erfordert bevorzugt saugfähige oder zumindest von Heißschmelzklebemasse benetzbare Trägermaterialien. Nichtbenetzende Trägeroberflächen müssen durch chemische oder physikalische Verfahren vorbehandelt werden. Dies kann durch zusätzliche Maßnahmen wie zum Beispiel Coronaentladung oder Beschichtung mit die Benetzung verbessernden Stoffen geschehen.

Mit dem aufgezeigten Druckverfahren kann die Größe und Form der Kalotten definiert festgelegt werden. Die für die Anwendung relevanten Klebkraftwerte, die die Qualität der erzeugten Produkte bestimmen, liegen bei sachgerechter Beschichtung in sehr engen Toleranzen. Der Basisdurchmesser der Kalotten kann von 10 µm bis 5000 µm gewählt werden, die Höhe der Kalotten von 20 µm bis ca. 2000 µm, bevorzugt 50 µm bis 1000 µm, wobei der Bereich kleiner Durchmesser für glatte Träger, der mit größerem Durchmesser und größerer Kalottenhöhe für rauhe oder stark porige Trägermaterialien vorgesehen ist.

Die Positionierung der Kalotten auf dem Träger wird durch die in weiten Grenzen variierbare Geometrie des Auftragswerkes, zum Beispiel Gravur- oder Siebgeometrie, definiert festgelegt. Mit Hilfe der aufgezeigten Parameter kann über einstellbare Größen das gewünschte Eigenschaftsprofil der Beschichtung, abgestimmt auf die verschiedenen Trägermaterialien und Anwendungen, sehr genau eingestellt werden.

Das Trägermaterial wird bevorzugt mit einer Geschwindigkeit von größer 2 m/min, bevorzugt 20 bis 220 m/min, beschichtet, wobei die Beschichtungstemperatur größer als die Erweichungstemperatur zu wählen ist.

Die Heißschmelzklebemasse kann mit einem Flächengewicht von größer 15 g/m$^2$, bevorzugt zwischen 20 g/m$^2$ und 300 g/m$^2$, ganz besonders bevorzugt zwischen 90 g/m$^2$ und 160 g/m$^2$, auf dem Trägermaterial aufgetragen sein.

Der prozentuale Anteil, der mit der Heißschmelzklebemasse beschichteten Fläche sollte mindestens 20 % betragen und kann bis zu ungefähr 95 % reichen, für spezielle Produkte bevorzugt 40 % bis 60 % sowie 70 % bis 95 %. Dieses kann gegebenenfalls durch Mehrfachapplikation erreicht werden, wobei gegebenenfalls auch Heißschmelzklebemassen mit unterschiedlichen Eigenschaften eingesetzt werden können.

Weiterhin hat es sich als vorteilhaft herausgestellt, den Klebstoff mit Gas zu beladen, wodurch sich eine Verbesserung der klebetechnischen Eigenschaften einstellt. So können sich beispielsweise ein besseres Anschmiegen und eine Erhöhung der initialen Klebkraft ergeben. Bevorzugt wird eine Beladung der Heißschmelzselbstklebemasse mit einem Gasanteil von mehr als 5 Vol.-%, bevorzugt von 20 Vol.-% bis 85 Vol.-%.

Die Kombination der Heißschmelzklebemasse und der partiellen Beschichtung sichert auf der einen Seite eine sichere Verklebung des medizinischen Produktes auf der Haut, auf der anderen Seite sind zumindest visuell erkennbare allergische oder mechanische Hautirritationen ausgeschlossen, auch bei einer Anwendung, die sich über mehrere Tage erstreckt.

Die Epilation entsprechender Körperregionen und der Massetransfer auf die Haut sind aufgrund der hohen Kohäsivität des Klebers vernachlässigbar, weil der Kleber nicht an Haut und Haar verankert, vielmehr ist die Verankerung der Selbstklebemasse auf dem Trägermaterial mit bis zu 12 N/cm (Probenbreite) für medizinische Anwendungen gut.

Durch die ausgeformten Sollbruchstellen in der Beschichtung werden Hautlagen beim Ablösen nicht mehr mit- oder gegeneinander verschoben. Das Nichtverschieben der Hautlagen und die geringere Epilation führen zu einem bisher nicht gekannten Grad der Schmerzfreiheit bei solchen stark klebenden Systemen. Das applizierte Trägermaterial zeigt gute propriorezeptive Wirkungen.

Je nach Trägermaterial und dessen Temperaturempfindlichkeit kann die Heißschmelzklebemasse direkt aufgetragen sein oder zuerst auf einen Hilfsträger aufgebracht und dann auf den endgültigen Träger transferiert werden. Auch ein nachträgliches Kalandern des beschichteten Produktes und/oder eine Vorbehandlung des Trägers, wie Coronabestrahlung, zur besseren Verankerung der Klebeschicht kann vorteilhaft sein.

Das mit der Klebemasse beschichtete Trägermaterial kann eine Luftdurchlässigkeit von größer 1 $cm^3/(cm^2*s)$, bevorzugt größer 15 $cm^3/(cm^2*s)$, ganz besonders bevorzugt größer 70 $cm^3/(cm^2*s)$, aufweisen, des weiteren eine Wasserdampfdurchlässigkeit von größer 500 $g/(m^2*24h)$, bevorzugt größer 1000 $g/(m^2*24h)$, ganz besonders bevorzugt größer 2000 $g/(m^2*24h)$.

Schließlich kann das Trägermaterial nach dem Beschichtungsvorgang mit einem klebstoffabweisenden Trägermaterial, wie silikonisiertes Papier, eingedeckt oder mit einer Wundauflage oder einer Polsterung versehen werden.

Besonders vorteilhaft ist, daß das selbstklebend ausgerüstete Trägermaterial sterilisierbar, bevorzugt γ-(gamma) sterilisierbar, ist. So sind besonders geeignet für eine nachträgliche Sterilisation Heißschmelzklebemassen auf Blockcopolymerbasis, welche keine Doppelbindungen enthalten. Dieses gilt ins besondere für Styrol-Butylen-Ethylen-Styrol-Blockcopolymerisate oder Styrol-Butylen-Styrol-Blockcopolymerisate. Es treten hierbei keine für die Anwendung signifikanten Änderungen in den Klebeeigenschaften auf.

Schließlich kann die Selbstklebemasse einen Wirkstoff enthalten. Bei den dotierten Selbstklebemassen sind insbesondere solche bevorzugt, die Stoffe freisetzen.

Das erfindungsgemäße Trägermaterial weist eine Klebkraft auf der Trägerrückseite von mindestens 0,5 N/cm auf, besonders eine Klebkraft zwischen 1 N/cm und 5 N/cm. Auf anderen Untergründen können höhere Klebkräfte erreicht werden.

Die hervorragenden Eigenschaften des erfindungsgemäßen, selbstklebend ausgerüsteten Trägermaterials legen die Verwendung für medizinische Produkte, insbesondere Pflaster, medizinische Fixierungen, Wundabdeckungen, orthopädische oder phlebologische Bandagen und Binden nahe.

Im folgenden soll das erfindungsgemäße selbstklebend ausgerüstete Trägermaterial mittels von Beispielen dargestellt werden, ohne die Erfindung unnötig einschränken zu wollen.

**Beispiel 1:**

Erfindungsgemäß wurde eine elastische selbstklebende Binde hergestellt, die aufgrund ihrer nachfolgend beschriebenen Eigenschaften zur Anwendung als funktioneller Verband dienen kann, wobei sich die Funktionelle Verbandtechnik an der Anatomie und der Biomechanik orientiert.

Die für diesen Verbandtyp verwendete Binde bestand aus einem elastischen Baumwollgewebe mit einer Höchstzugkraft von größer 80 N/cm und einer Höchstzugkraft-Dehnung von größer 100 %. Die Dehnbarkeit bei 10 N/cm des unbeschichteten Trägermaterials betrug 75 % und wurde durch die Beschichtung nicht wesentlich verringert.

Die Selbstklebemasse wurde im Thermosiebdruck auf den Träger appliziert, wobei es sich um einen Heißschmelzklebemasse handelte.

Diese Heißschmelzklebemasse setzte sich wie folgt zusammen:

- ein A-B/A-B-A Blockcopolymer, welches aus harten und weichen Segmenten bestand, mit einem Verhältnis der A-B-A zur A-B von 2:1 und einem Styrolgehalt im Polymer von 13 Mol.-%; der Anteil an der Selbstklebemasse betrug 40 Gew.-% (Kraton G)
- ein Paraffinkohlenwasserstoffharz mit einem Anteil an der Klebmasse von 52 Gew.-%
- Kohlenwasserstoffharze mit einem Anteil von 7,5 Gew.-% (Super Resin HC 140)
- ein Alterungsschutzmittel mit einem Anteil von weniger als 0,5 Gew.-% (Irganox).

Die eingesetzten Komponenten wurden in einem Thermomischer bei 175 °C homogenisiert.

Der Erweichungspunkt dieser Selbstklebemasse betrug 95 °C (DIN 52011), und die Klebmasse zeigte eine Viskosität von 2100 mPas bei 150 °C (DIN 53018, Brookfield DV II, Sp. 21). Die Glasübergang betrug nach oben angegebener Methode -8 °C.

Die direkte Beschichtung erfolgte mit 50 m/min bei einer Temperatur von 130 °C. Das Trägermaterial wurde mit 120 g/m$^2$ punktförmig beschichtet, wobei eine 14 Meshsieb-Schablone mit einer Siebstärke von 300 µm verwendet wurde.

Die nach diesem Verfahren hergestellte Binde zeigte eine gute Ablösefähigkeit durch Dehnung in Orientierung der Hautoberfläche sowie eine gute Luft- und Wasserdampfdurchlässigkeit. Aufgrund der hohen Scherstabilität des Schmelzhaftklebers wurde eine ausreichend Stabilisierung und eine gute propriorezeptive Wirkung festgestellt. Es wurden keine Hautirritationen und eine vernachlässigbar geringe Epilation nach dem Abnehmen der Binde beobachtet.

Eine Vergleichsuntersuchung auf Stahl zeigte, daß die zum Ablösen der Bandage benötigte Scherkraft ungefähr nur noch ein Drittel der Scherkraft beträgt, die ein vollflächig beschichtetes Bandagenmaterial bei gleichem Masseauftrag aufweist.


### Beispiel 2

Es wurde ein durch Dehnung wiederablösbares Filtermaterial hergestellt, welches auf einem elastischen Vlies basierte. Bei diesem Filtermaterial wurde die filternde Wirkung durch die partielle Beschichtung erhalten.

Das Filtermaterial bestand aus einem handelsüblichen Filtervliesstoff mit thermoplastischen Eigenschaften. Das Vlies hatte eine Höchstzugkraftdehnung von 250 % und eine Dehnung bei einer Belastung von 10 N/cm von 150 %.

Bei dem Blockcopolymeren handelte es sich um ein Styrol-Ethylen-Butylen-Styrol-Blockcopolymerisat, welches mit Paraffinkohlenwasserstoffenwachs versetzt worden ist. Das Verhältnis war ein Teil Polymer auf ein Teil Paraffinkohlenwasserstoff. Zu dieser Mischung wurden 10 % Polystyrolharz (Amoco 18240) gegeben. Der Kleber enthielt ein Prozent Irganox, ein Alterungsschutzmittel (β-(3,5 Di-t.butyl-4-hydroxyphenyl)-propionsäure-n-octadecylester)), und weitere Kohlenwasserstoffharze und Fettsäureester, welche im Gesamtkleber nur zu geringen Mengen enthalten waren. Der Erweichungspunkt dieser Selbstklebemasse betrug 100 °C (DIN 52011) und die Glasübergangstemperatur, bestimmt nach oben genannter Methode, -6 °C.

Mit einer 40 Mesh Siebschablone mit einem Durchlaß von 20 % konnte ein Masseauftrag von 20g/m$^2$ erzielt werden.

Das so hergestellte Filtermaterial wies eine hohe Luftdurchlässigkeit auf, und zwar mehr als 85 cm$^3$/(cm$^2$*s).

Die elastische adhäsive Filtervorrichtung erwies sich insbesondere bei häufig wechselnden Einsatzorten aufgrund ihrer schnellen Entfernbarkeit als vorteilhaft. Durch die ganzseitige partielle Beschichtung ergab sich ebenfalls eine schnelle und einfache Montage dieser Filtermaterialien. Die Scherkraft betrug 6 N/cm.


**Patentansprüche**

1. Verfahren zur Verwendung eines zumindest einseitig selbstklebend ausgerüsteten Trägermaterials **gekennzeichnet durch** die Schritte

   - Herstellung medizinischer Produkte,
   - Applikation dieser auf der menschlichen Haut und
   - Ablösung dieser **durch** Ziehen in Richtung der Verklebungsebene vom Untergrund,

   wobei das Trägermaterial partiell mit einer Selbstklebemasse beschichtet ist, eine Klebkraft auf der Trägerrückseite von mindestens 0,5 N/cm und bei einer Zugbelastung von 10 N/cm eine Dehnung von 15% bis 3000% aufweist.

2. Verfahren nach Anspruch 1, **dadurch gekennzeichnet, dass** das Trägermaterial bei einer Zugbelastung von 10 N/cm eine Dehnung von größer 20% bis 1000% aufweist.

3. Verfahren nach Anspruch 1 oder 2, **dadurch gekennzeichnet, dass** die Selbstklebemasse eine Heißschmelzklebemasse ist, die eine dynamisch-komplexe Glasübergangstemperatur bei einer Frequenz von 0,1 rad/s von weniger als 5 °C, bevorzugt von -3 °C bis -30 °C, besonders bevorzugt von -9 °C bis -25 °C, aufweist.

4. Verfahren nach Anspruch 3, **dadurch gekennzeichnet, dass** die Heißschmelzklebemasse auf Blockcopolymerbasis aufgebaut ist, insbesondere A-B- oder A-B-A-Blockcopolymere oder deren Mischungen, wobei Phase A vornehmlich Polystyrol oder dessen Derivate und Phase B Ethylen, Propylen, Butylen, Butadien, Isopren oder deren Mischungen, hierbei besonders bevorzugt Ethylen und Butylen oder deren Mischungen, sind.

**5.** Verfahren nach einem der Ansprüche 3 oder 4, **dadurch gekennzeichnet, dass** die Heißschmelzklebemasse besteht aus

- 10 Gew.-% bis 90 Gew.-% Blockcopolymeren,
- 5 Gew.-% bis 80 Gew.-% Klebrigmachern wie Ölen, Wachsen, Harzen und deren Mischungen, bevorzugt Mischungen aus Harzen und Ölen,
- gegebenenfalls weniger als 60 Gew.-% Weichmachern,
- weniger als 15 Gew.-% Additiven
- weniger als 5 Gew.-% Stabilisatoren.

**6.** Verfahren nach einem der Ansprüche 3 bis 5, **dadurch gekennzeichnet, dass** die Heißschmelzklebemasse durch Rasterdruck, Thermosiebdruck oder Tiefdruck aufgebracht wird.

**7.** Verfahren nach einem der Ansprüche 3 bis 6, **dadurch gekennzeichnet, dass** die Heißschmelzklebemasse in Form von polygeometrischen Kalotten auf das Trägermaterial gebracht wird.

**8.** Verfahren nach einem der Ansprüche 3 bis 7, **dadurch gekennzeichnet, dass** die Heißschmelzklebemasse mit einem Flächengewicht von größer 15 g/m$^2$, bevorzugt zwischen 20 g/m$^2$ und 300 g/m$^2$, ganz besonders bevorzugt zwischen 90 g/m$^2$ und 160 g/m$^2$, auf dem Trägermaterial beschichtet wird.

**9.** Verfahren nach einem der vorhergehenden Ansprüche, **dadurch gekennzeichnet, dass** das beschichtete Trägermaterial eine Luftdurchlässigkeit von größer 1 cm$^3$/(cm$^2$*s), bevorzugt größer 15 cm$^3$/(cm$^2$*s), ganz besonders bevorzugt größer 70 cm$^3$/(cm$^2$*s), und/oder eine Wasserdampfdurchlässigkeit von größer 500 g/(m$^2$*24h), bevorzugt größer 1000 g/(m$^2$*24h), ganz besonders bevorzugt größer 2000 g/(m$^2$*24h) aufweist.

**10.** Verfahren nach einem der vorhergehenden Ansprüche, **dadurch gekennzeichnet, dass** das Trägermaterial eine Klebkraft auf der Trägerrückseite zwischen 1 N/cm und 5 N/cm aufweist.

**11.** Verfahren nach einem der vorhergehenden Ansprüche, **dadurch gekennzeichnet, dass** die Heißschmelzklebemasse einen Gasanteil von mehr als 5 Vol.-% aufweist, bevorzugt von 20 Vol.-% bis 85 Vol.-% aufweist.

**12.** Verfahren nach einem der vorhergehenden Ansprüche, **dadurch gekennzeichnet, daß** die medizinischen Produkte orthopädische oder phlebologische Bandagen und Binden sind.

**13.** Verfahren nach einem der vorhergehenden Ansprüche, **dadurch gekennzeichnet, dass** das partiell beschichtete Trägermaterial nach der Applikation eingedeckt oder mit einer Wundauflage oder Polsterung versehen wird.

**14.** Verfahren nach einem der vorhergehenden Ansprüche, **dadurch gekennzeichnet, dass** das partiell beschichtete Trägermaterial sterilisiert wird, bevorzugt γ (gamma)-sterilisiert wird.

**15.** Verfahren nach einem der vorstehenden Ansprüche, **dadurch gekennzeichnet, dass** die Selbstklebemasse einen Wirkstoff enthält.

**Claims**

**1.** Method of using an at least one-sidedly self-adhesively treated backing material **characterized by** the following steps

- production of medical products,
- application of said products to the human skin and
- detachment of said products from the substrate by pulling in the direction of the bond plane,

where the backing material is coated partially with a self-adhesive composition, has a bond strength to the reverse of the backing of at least 0.5 N/cm and which under a tensile load of 10 N/cm has an extension of from 15% to 3000%.

**2.** Method according to Claim 1, **characterized in that** under a tensile load of 10 N/cm the backing material has an extension of greater than 20% up to 1000%.

3. Method according to Claim 1 or 2, **characterized in that** the self-adhesive composition is a hotmelt adhesive composition having a dynamic-complex glass transition temperature with a frequency of 0.1 rad/s of less than 5°C, preferably from -3 to -30°C and, with particular preference, from -9 to -25°C.

4. Method according to Claim 3, **characterized in that** the hotmelt adhesive composition is built up on the basis of block copolymers, especially A-B or A-B-A block copolymers or mixtures thereof, with phase A being principally polystyrene or its derivatives and phase B ethylene, propylene, butylene, butadiene, isoprene or mixtures thereof, with particular preference being given to ethylene and butylene or their mixtures.

5. Method according to either of Claims 3 and 4, **characterized in that** the hotmelt adhesive composition consists of

   - from 10 to 90% by weight of block copolymers,
   - from 5 to 80% by weight of tackifiers such as oils, waxes, resins and mixtures thereof, preferably mixtures of resins and oils,
   - if desired, less than 60% by weight of plasticizers,
   - less than 15% by weight of additives,
   - less than 5% by weight of stabilizers.

6. Method according to one of Claims 3 to 5, **characterized in that** the hotmelt adhesive composition is applied by halftone printing, thermal screen printing or intaglio printing.

7. Method according to one of Claims 3 to 6, **characterized in that** the hotmelt adhesive composition is applied in the form of polygeometric domes to the backing material.

8. Method according to one of Claims 3 to 7, **characterized in that** the hotmelt adhesive composition has been coated onto the backing material in a weight per unit area of more than 15 g/m$^2$, preferably between 20 and 300 g/m$^2$ and, with very particular preference between 90 and 160 g/m$^2$.

9. Method according to one of the preceding claims, **characterized in that** the coated backing material has an air permeability of greater than 1 cm$^3$/(cm$^2$*s), preferably greater than 15 cm$^3$/(cm$^2$*s) and, with very particular preference, greater than 70 cm$^3$/(cm$^2$*s) and/or a water vapour permeability of greater than 500 g/(m$^2$*24h), preferably greater than 1000 g/(m$^2$*24h), and, with very particular preference, greater than 2000 g/(m$^2$*24h).

10. Method according to one of the preceding claims, **characterized in that** the backing material has a bond strength to the reverse of the backing of between 1 and 5 N/cm.

11. Method according to one of the preceding claims, **characterized in that** the hotmelt adhesive composition has a gas content of more than 5% by volume, preferably from 20 to 85% by volume.

12. Method according to one of the preceding claims, **characterized in that** the medical products are orthopaedic or phlebological bandages and dressings.

13. Method according to one of the preceding claims, **characterized in that** the partially coated backing material is lined after application or provided with a wound pad or padding.

14. Method according to one of the preceding claims, **characterized in that** the partially coated backing material is sterilized, preferably by means of γ (gamma) radiation.

15. Method according to one of the preceding claims, **characterized in that** the self-adhesive composition includes an active substance.

**Revendications**

1. Procédé d'utilisation d'un matériau de support ayant fait l'objet d'un traitement autocollant sur au moins un côté, **caractérisé par** les étapes :

- production de produits médicinaux,
- application de ceux-ci sur la peau humaine et
- détachement de ceux-ci du substrat en tirant dans la direction du plan d'adhésion,
- le matériau de support étant partiellement revêtu d'une composition autocollante, présentant une force d'adhésion sur le revers du support d'au moins 0,5 N/cm et un allongement de 15 % à 3 000 % pour une charge de traction de 10 N/cm.

2. Procédé selon la revendication 1, **caractérisé en ce que** le matériau de support présente un allongement de plus de 20 % à 1 000 % pour une charge de traction de 10 N/cm.

3. Procédé selon la revendication 1 ou 2, **caractérisé en ce que** la composition autocollante est une composition adhésive thermofusible qui présente une température de transition vitreuse en dynamique complexe, à une fréquence de 0,1 rad/s, inférieure à 5°C, de préférence de -3°C à -30°C, particulièrement préférablement de -9°C à -25°C.

4. Procédé selon la revendication 3, **caractérisé en ce que** la composition adhésive thermofusible est formulée à base de copolymères blocs, en particulier de copolymères blocs A-B ou A-B-A ou leurs mélanges, la phase A étant constituée principalement de polystyrène ou de ses dérivés et la phase B étant constituée d'éthylène, de propylène, de butylène, de butadiène, d'isoprène ou de leurs mélanges, l'éthylène et le butylène ou leurs mélanges étant en l'occurrence particulièrement préférés.

5. Procédé selon l'une quelconque des revendications 3 ou 4, **caractérisé en ce que** la composition adhésive thermofusible est constituée de :

- 10 % en poids à 90 % en poids de copolymères blocs,
- 5 % en poids à 80 % en poids d'agents collants tels que des huiles, des cires, des résines et leurs mélanges, de préférence des mélanges de résines et d'huiles,
- éventuellement moins de 60 % en poids de plastifiants,
- moins de 15 % en poids d'additifs,
- moins de 5 % en poids d'agents stabilisants.

6. Procédé selon l'une quelconque des revendications 3 à 5, **caractérisé en ce que** la composition adhésive thermofusible est appliquée par impression en demi-teintes, thermosérigraphie ou héliogravure.

7. Procédé selon l'une quelconque des revendications 3 à 6, **caractérisé en ce que** la composition adhésive thermofusible est appliquée sous forme de calottes polygéométriques sur le matériau de support.

8. Procédé selon l'une quelconque des revendications 3 à 7, **caractérisé en ce que** la composition adhésive thermofusible est appliquée en couche sur le matériau de support avec un poids surfacique supérieur à 15 g/m$^2$, de préférence compris entre 20 g/m$^2$ et 300 g/m$^2$, tout particulièrement préférablement entre 90 g/m$^2$ et 160 g/m$^2$.

9. Procédé selon l'une quelconque des revendications précédentes, **caractérisé en ce que** le matériau de support revêtu présente une perméabilité à l'air supérieure à 1 cm$^3$/(cm$^2$*s), de préférence supérieure à 15 cm$^3$/(cm$^2$*s), tout particulièrement préférablement supérieure à 70 cm$^3$/(cm$^2$*s), et/ou une perméabilité à la vapeur d'eau supérieure à 500 g/(m$^2$*24h), de préférence supérieure à 1 000 g/(m$^2$*24h), tout particulièrement préférablement supérieure à 2 000 g/(m$^2$*24h).

10. Procédé selon l'une quelconque des revendications précédentes, **caractérisé en ce que** le matériau de support présente une force d'adhésion sur le revers du support comprise entre 1 N/cm et 5 N/cm.

11. Procédé selon l'une quelconque des revendications précédentes, **caractérisé en ce que** la composition adhésive thermofusible présente une proportion gazeuse supérieure à 5 % en volume, de préférence de 20 % en volume à 85 % en volume.

12. Procédé selon l'une quelconque des revendications précédentes, **caractérisé en ce que** les produits médicinaux sont des bandages et des pansements orthopédiques ou phlébologiques.

13. Procédé selon l'une quelconque des revendications précédentes, **caractérisé en ce que** le matériau de support partiellement revêtu est recouvert ou pourvu d'un tampon pour plaies ou d'un rembourrage après l'application.

14. Procédé selon l'une quelconque des revendications précédentes, **caractérisé en ce que** le matériau de support partiellement revêtu est stérilisé, de préférence par stérilisation $\gamma$ (gamma).

15. Procédé selon l'une quelconque des revendications précédentes, **caractérisé en ce que** la composition autocollante contient un ingrédient actif.